(19) Europäisches Patentamt
European Patent Office
Office européen des brevets

(11) EP 2 921 186 B1

(12) **EUROPEAN PATENT SPECIFICATION**

(45) Date of publication and mention of the grant of the patent:
**08.05.2019 Bulletin 2019/19**

(21) Application number: **15159360.5**

(22) Date of filing: **17.03.2015**

(51) Int Cl.:
***A61L 27/16*** *(2006.01)*
***B29C 43/10*** *(2006.01)*
***B29C 71/04*** *(2006.01)*
***B29B 11/14*** *(2006.01)*
***B29B 13/08*** *(2006.01)*

(54) **ANNEALING METHOD FOR CROSS-LINKED POLYETHYLENE**

VERFAHREN ZUM AUSGLÜHEN VON VERNETZTEM POLYETHYLEN

PROCÉDÉ DE RECUISSON DE POLYÉTHYLÈNE RÉTICULÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.03.2014 US 201414221995**

(43) Date of publication of application:
**23.09.2015 Bulletin 2015/39**

(73) Proprietor: **Howmedica Osteonics Corp.**
**Mahwah, NJ 07430 (US)**

(72) Inventors:
- **Song, Lin**
  **Wayne, NJ New Jersey 07470 (US)**
- **Lawrynowicz, Daniel E.**
  **Monroe, NY New York 10950 (US)**
- **Le, Kim-phuong Nguyen**
  **Dunellen, NJ New Jersey 08812 (US)**
- **Tenhuisen, Kevor Shane**
  **Boulder, CO Colorado 80301 (US)**

(74) Representative: **Valea AB**
**Box 1098**
**405 23 Göteborg (SE)**

(56) References cited:
**EP-A1- 1 369 135**
**WO-A2-2008/124825**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 921 186 B1

Printed by Jouve, 75001 PARIS (FR)

## Description

BACKGROUND OF THE INVENTION

This invention relates to medical implants formed of a polymeric material such as ultra-high molecular weight polyethylene (UHMWPE), with superior properties such as, superior oxidation and wear resistance produced by a sequential irradiation and annealing process, further including a hot isostatic pressing step performed on a UHMWPE preform. A preform may be an UHMWPE rod, bar, plate or portion thereof consolidated from an UHMWPE resin.

Various polymer systems have been used for the preparation of artificial prostheses for biomedical use, particularly orthopedic applications. Among them, ultra-high molecular weight polyethylene is widely used for articulation surfaces in artificial knee, hip, and other joint replacements. Ultra-high molecular weight polyethylene (UHMWPE) has been defined as those linear polyethylenes which have a relative viscosity of 2.3 or greater at a solution concentration of 0.05% at 135°C in decahydronaphthalene. The nominal weight - average molecular weight is at least 400,000 and up to 10,000,000 and usually from three to six million. The manufacturing process begins with the polymer being supplied as fine resin powder which is consolidated into various forms, such as rods, bars and slabs, using ram extrusion injection molding or compression molding. Afterwards, the consolidated rods or slabs are machined into the final shape of the orthopedic implant components. Alternatively, the component can be produced by compression or injection molding of the UHMWPE resin powder.

All components must then go through a sterilization procedure prior to use, but usually after being packaged. There exists several sterilization methods which can be utilized for medical applications, such as the use of ethylene oxide, gas plasma, heat, or radiation. However, applying heat to a packaged polymeric medical product can destroy either the integrity of the packaging material (particularly the seal, which prevents bacteria from going into the package after the sterilization step) or the product itself.

It has been recognized that regardless of the radiation type, the high energy beam causes generation of free radicals in polymers during radiation. It has also been recognized that the amount or number of free radicals generated is dependent upon the radiation dose received by the polymers and that the distribution of free radicals in the polymeric implant depends upon the geometry of the component, the type of polymer, the dose rate, and the type of radiation beam. The generation of free radicals can be described by the following reaction (which uses polyolefin and gamma ray irradiation for illustration):

gamma rays

Polyolefin ---------------- r· where r· are primary free radicals * (1)

*(through C-C chain scission or C-H scission)

Depending on whether or not oxygen is present, primary free radicals r· will react with oxygen and the polymer according to the following reactions as described in "Radiation Effects on Polymers," edited by Roger L. Clough and Shalaby W. Shalaby, published by American Chemical Society, Washington, D.C., 1991.

<u>In the presence of oxygen</u>

$O_2$

r·-----------$rO_2$ (2)

$rO_2$ + polyolefin -------rOOH + P· (3)

P·+$O_0$ ---------------- $P0_2$. (4)

$O_2$

$P0_2$·+ polyolefin ---- POOH + P·--------$P0_2$· (5)

$rO_2$., $P0_2$.------ Some chain scission products (6)

room temperature

rOOH, POOH -------------- free radicals, rOH, POH (7)

P· + $P0_2$ ------ POOP (ester cross-links) (8)

2 P·-------------P-P (C-C cross-links) (9)

In radiation in air, primary free radicals r· will react with oxygen to form peroxyl free radicals $r0_2$·, which then react with polyolefin (such as UHMWPE) to start the oxidative chain scission reactions (reactions 2 through 6). Through these reactions, material properties of the plastic, such as molecular weight, tensile and wear properties, are degraded.

It has been found that the hydroperoxides (rOOH and POOH) formed in reactions 3 and 5 will slowly break down as shown in reaction 7 to initiate post-radiation degradation. Reactions 8 and 9 represent termination steps of free radicals to form ester or carbon-carbon cross-links. Depending on the type of polymer, the extent of reactions 8 and 9 in relation to reactions 2 through 7 may vary. For irradiated UHMWPE, a value of 0.3 for the ratio of chain scission to cross-linking has been obtained, indicating that even though cross-linking is a dominant mechanism, a significant amount of chain scission occurs in irradiated polyethylene.

By applying radiation in an inert atmosphere, since there is no oxidant present, the primary free radicals r· or secondary free radicals P· can only react with other neighboring free radicals to form carbon-carbon cross-links, according to reactions 10 through 12 below. If all the free radicals react through reactions 10 through 12, there will be no chain scission and there will be no molecular weight degradation. Furthermore, the extent of cross-linking is increased over the original polymer prior to irradiation. On the other hand, if not all the free radicals formed are combined through reactions 10, 11 and 12, then some free radicals will remain in the plastic component.

In an Inert Atmosphere

r· + polyolefin ----------- P· (10)

2 r· ---------- r-r (C-C cross-linking) (11)

2 P· ---------- P-P (C-C cross-linking) (12)

It is recognized that the fewer the free radicals, the better the polymer retains its physical properties over time. The greater the number of free radicals, the greater the degree of molecular weight and polymer property degradation will occur. Applicant has discovered that the extent of completion of free radical cross-linking reactions is dependent on the reaction rates and the time period given for reaction to occur.

UHMWPE is commonly used to make prosthetic joints such as artificial hip joints. In recent years, it has been found that tissue necrosis and interface osteolysis may occur in response to UHMWPE wear debris. For example, wear of acetabular cups of UHMWPE in artificial hip joints may introduce microscopic wear particles into the surrounding tissues.

Improving the wear resistance of the UHMWPE socket and, thereby, reducing the rate of production of wear debris may extend the useful life of artificial joints and permit them to be used successfully in younger patients. Consequently, numerous modifications in physical properties of UHMWPE have been proposed to improve its wear resistance.

It is known in the art that ultrahigh molecular weight polyethylene (UHMWPE) can be cross-linked by irradiation with high energy radiation, for example gamma radiation, in an inert atmosphere or vacuum. Exposure of UHMWPE to gamma irradiation induces a number of free-radical reactions in the polymer. One of these is cross-linking. This cross-linking creates a 3-dimensional network in the polymer which renders it more resistant to adhesive wear in multiple directions. The free radicals formed upon irradiation of UHMWPE can also participate in oxidation which reduces the molecular weight of the polymer via chain scission, leading to degradation of physical properties, embrittlement and a significant increase in wear rate. The free radicals are very long-lived (greater than eight years), so that oxidation continues over a very long period of time resulting in an increase in the wear rate as a result of oxidation over the life of the implant.

Sun et al. U.S. Patent No. 5,414,049 broadly discloses the use of radiation to form free radicals and heat to form cross-links between the free radicals prior to oxidation.

Hyon et al. U.S. Patent No. 6,168,626 relates to a process for forming oriented UHMWPE materials for use in artificial joints by irradiating with low doses of high-energy radiation in an inert gas or vacuum to cross-link the material to a low degree, heating the irradiated material to a temperature at which compressive deformation is possible, preferably to a temperature near the melting point or higher, and performing compressive deformation followed by cooling and solidifying the material. The oriented UHMWPE materials have improved wear resistance. Medical implants may be machined from the oriented materials or molded directly during the compressive deformation step. The anisotropic nature of the oriented materials may render them susceptible to deformation after machining into implants.

Salovey et al. U.S. Patent No. 6,228,900 relates to a method for enhancing the wear-resistance of polymers, including UHMWPE, by cross-linking them via irradiation in the melt.

Saum et al. U.S. Patent No. 6,316,158 relates to a process for treating UHMWPE using irradiation followed by thermally treating the polyethylene at a temperature greater than 150°C to recombine cross-links and eliminate free radicals.

Several other prior art patents attempt to provide methods which enhance UHMWPE physical properties. European Patent Application 0 177 522 81 relates to UHMWPE powders being heated and compressed into a homogeneously melted crystallized morphology with no grain memory of the UHMWPE powder particles and with enhanced modulus and strength. U.S. Patent No. 5,037,928 relates to a prescribed heating and cooling process for preparing a UHMWPE exhibiting a combination of properties including a creep resistance of less than 1% (under exposure to a temperature of 23°C and a relative humidity of 50% for 24 hours under a compression of 1000 psi, i.e. 7 MPa) without sacrificing tensile and flexural properties. U.K. Patent Application GB 2 180 815 A relates to a packaging method where a medical device which is sealed in a sterile bag, after radiation/sterilization, is hermetically sealed in a wrapping member of oxygen-impermeable material together with a deoxidizing agent for prevention of post-irradiation oxidation.

U.S. Patent No. 5,153,039 relates to a high density polyethylene article with oxygen barrier properties. U.S. Patent No. 5,160,464 relates to a vacuum polymer irradiation process. European Patent Application 03253363.0, publication no. EP 1369135 A1, relates to a method of producing a highly cross-linked polyethylene material, especially an ultra-high molecular weight polyethylene (UHMWPE), utilizing a sequential irradiation and annealing process. International Patent Application PCT/US2008/059909, publication No. WO 2008/124825 A2, relates to a method for processing UHMWPE for use in medical applications, the method comprising combining UHMWPE with an antioxidant to form a blend; processing the blend to consolidate; preheating the consolidated blend; and irradiating the consolidated blend while maintaining the consolidated blend at a temperature below the melting point of the consolidated blend.

## BRIEF SUMMARY OF THE INVENTION

The present invention relates to a method for providing a polymeric material, such as UHMWPE, with mechanical properties such as, superior oxidation resistance, mechanical strength and wear properties. The method involves using a series of relatively low doses of radiation with an annealing process after each dose, further comprising a hot isostatic pressing step performed before, after or between the sequential irradiation and cross-linking steps.

A sequentially cross-linking process used with the present invention is taught in U.S. Patent Nos. 7,517,917, 7,714,036, 8,030,370, and 8,324,291. It has now been unexpectedly found that the addition of a hot isostatic pressing step at a pressure of 2,000 to 30,000 psi, i.e. 14 to 207 MPa, and a temperature between 150°C and 170°C for 8 hours provides even more improvement in physical properties than that achieved by the sequential cross-linking of the prior art taught in the ’017, ’036, ’370 and ’291 patents listed above.

As stated above, UHMWPE polymer is very stable and has very good resistance to aggressive media except for strong oxidizing acids. Upon irradiation, free radicals are formed which cause UHMWPE to become activated for chemical reactions and physical changes. Possible chemical reactions include reacting with oxygen, water, body fluids, and other chemical compounds while physical changes include density, crystallinity, color, and other physical properties. In the present invention, the sequential radiation and annealing process greatly improves the physical properties of UHMWPE when compared to applying the same total radiation dose in one step. Furthermore, this process does not employ stabilizers, antioxidants, or any other chemical compounds which may have potentially adverse effects in biomedical or orthopedic applications.

It is also known that at relatively low dose levels (<50 kGy) of irradiation residual free radicals are mostly trapped in the crystalline region while most free radicals crosslink in the amorphous region. There is a steep free radical concentration gradient across the crystalline-amorphous boundary, which provides a significant driving force for free radicals to diffuse into the amorphous region where they can crosslink upon subsequent annealing. However, if the polyethylene is allowed to continuously accumulate higher radiation doses without interruptive annealing, molecules in the amorphous region become more and more stiffened due to increased crosslinking. As a result, the amorphous region traps more and more free radicals. This leads to a diminished free radical gradient across the crystalline-amorphous boundary, thereby reducing the driving force for free radical diffusion upon subsequent annealing. By limiting the incremental dose to below 50 kGy and preferably below 35 kGy and following with annealing, a relatively higher free radical diffusion

driving force can be maintained, allowing a more efficient free radical reduction upon annealing. If higher radiation doses are used, there could be cross-linking at the chain folded crystal surfaces. This could hamper the movement of free radicals from the crystal to the amorphous regions.

It has been found that polyethylene crystallinity increases continuously with increasing radiation-doses due to chain-scission (approximately 55% before radiation, increasing to 60% at 30 kGy, and to 65% at 100 kGy).

As the crystallinity increases with increasing dose of radiation, more residual free radicals are created and stored in the extra crystalline regions, which makes it increasingly more difficult to eliminate free radicals by annealing below the melt temperature. However, treating above the melting temperature (re-melting) significantly alters the crystallinity and crystal morphology which leads to significant reduction in mechanical properties such as yield strength and ultimate tensile strength and creep resistance and these properties are important for the structural integrity of the implant.

An orthopedic preformed material such as a rod, bar or compression molded sheet or plate for the subsequent production of a medical implant such as an acetabular or tibial implant with improved wear resistance is made from a polyethylene material cross-linked at least twice by irradiation and thermally treated by annealing after each irradiation. The material is cross-linked by a total radiation dose of from about 20 kGy to 1 MGy and preferably between 50 kGy and 100 kGy. The incremental dose for each irradiation is between about 20 kGy and about 50 kGy. The weight average molecular weight of the material is over 400,000.

The annealing takes place at a temperature greater than 25°C, preferably between 110°C and 135°C but less than the melting point. Generally, the annealing takes place for a time and temperature selected to be at least equivalent to heating the irradiated material at 50°C for 144 hours as defined by Arrenhius' equation 14. The material is heated for at least about 4 hours and then cooled to room temperature for the subsequent irradiation in the series.

By limiting the incremental dose to below 50 kGy and preferably below 35 kGy and following with annealing, the crystallinity will fluctuate between 55% and 60% (instead of 55-65%) and hence both the amount of chain-scission and residual free-radical concentration can be significantly reduced.

The polyethylene of the present invention may be in the form of a preformed rod such as an extruded rod or sheet with a subsequent production of a medical implant with improved wear resistance. The preformed rod or sheet is cross-linked at least twice by irradiation and thermally treated by annealing after each radiation. The incremental dose for each radiation is preferably between about 20 and 50 kGy with the total dose between 20 kGy and 1MGy and preferably between50 and 100 kGy.

After each irradiation, the preformed material is annealed either in air or in an inert atmosphere at a temperature of greater than 25°C and preferably less than 135°C or the melting point. Preferably, the annealing takes place for a time and temperature selected to be at least equivalent to heating the irradiated material at 50°C for 144 hours as defined by Arrenhius' equation (14). Generally, each heat treatment lasts for at least 4 hours and preferably about 8 hours.

Before or after the irradiation and annealing steps the preformed rod, bar, plate or sheet is hot isostatically pressed as described above to further improve mechanical properties such as the IZOD impact strength.

The preformed polyethylene material is then machined into a medical implant or other device. If the irradiation process occurred in air, then the entire outer skin to about 2 mm deep is removed from the preform prior to machining the medical implant or other device. If the process was done in a vacuum or an inner atmosphere such as nitrogen, then the outer skin may be retained.

Various aspects of the present invention are achieved by a method for making an ultra-high molecular weight polyethylene (UHMWPE) medical implant which includes obtaining a preform consolidated from UHMWPE resin to form a block or rod. This first consolidation step may be done by compression molding or extrusion (HIP). Thereafter the consolidated UHMWPE is hot isostatically pressed at a temperature between 150°C and 170°C at a pressure of between 2,000 and 30,000 psi, i.e. 14 to 207 MPa. This is done prior to irradiation as a second consolidation step. The advantage to performing the second consolidation prior to irradiation is that the polymer chains are not cross-linked, therefore more mobile, therefore more easily consolidated. As a result, better consolidation results can be obtained if consolidating non-cross-linked material. Prior art processes such as that of U.S. Patent No. 5,037,928 resulted in growth of the crystalline structures which in turn results in poor fatigue properties and wear properties. The two-step consolidation process of the present invention does not significantly impact crystalline grain size. The preform is then irradiated in a solid state to a total radiation dose of20 to 100 kGy. The irradiated UHMWPE preform is heated to a temperature of about 110°C to about 130°C for between 2 to 10 hours. The irradiated and heated preform is cooled to or below 50°C and thereafter a medical implant is formed from the preform.

The hot isostatic pressing of the preform occurs prior to irradiating the UHMWPE preform. The hot isostatic pressing occurs at a temperature between 150°C and 170°C.

The UHMWPE perform may be consolidated by a method selected from the group consisting of direct compression molding, extrusion and injection molding. Preferably the UHMWPE perform is irradiated at least two times and heated after each irradiation to below the melting temperature followed by cooling after each heating. Preferably the cooling of the preform to or below 50°C is done at a rate equal to or less than 2°C/min. More preferably the rate is less than 0.07°C/min. Preferably the heating temperature after irradiation for the UHMPWE is between 110°C and 130°C.

Aspects of the invention can also be achieved by a method of making an ultra-high molecular weight polyethylene (UHMWPE) which includes obtaining a preform, such as a sheet, rod or block, consolidated from a UHMWPE resin. After consolidation, the UHMWPE perform is irradiated in a solid state at a radiation dose of 20 to 100 kGy. A hot isostatic pressing of the UHMWPE preform then takes place at a temperature between 150°C and 170°C and a pressure of between 2,000 and 30,000 psi, i.e. 14 to 207 MPa, for at least about 8 hours. The irradiated preform is heated to a temperature of 110°C to 130°C for at least about 2 hours. The heated preform is cooled to a temperature to or below 50° prior to making a medical implant from the UHMWPE.

The hot isostatic pressing of the preform is at a temperature between 150°C and 170°C. The hot isostatic pressing of the preform is at a pressure between 2,000 psi and 30,000 psi, i.e. 14 to 207 MPa. The UHMWPE perform may be first consolidated by a method selected from the group consisting of direct compression molding, extrusion and injection molding. Preferably the hot isostatic pressing second consolidation is done prior to irradiating. The UHMWPE perform is preferably irradiated at least two times and heated between 110°C and 130°C for about eight hours after each irradiation followed by cooling after each heating.

The cooling is to or below 50°C and is done at a rate equal to or less than 2°C/min. The rate may be less than 0.07°C/min. Aspects of the invention may be achieved by a method for making an ultra-high molecular weight polyethylene (UHMWPE) medical implant including hot isostatically pressing (Hipping) a UHMWPE preform at a temperature between 150°C and 170°C at a pressure of between 2,000 and 30,000 psi, i.e. 14 to 207 MPa. After the HIP process, the UHMWPE preform may be irradiated at least three times to a total dose of5 to 100 kGy. The irradiated preform may be heated after each irradiation for at least about 8 hours at a temperature from 110°C to 130°C followed by cooling the UHMWPE preform after each irradiation and heating. The hot isostatic pressing of the preform occurs a single time prior to irradiating the UHMWPE preform. The hot isostatic pressing occurs at a temperature between 150°C and 170°C.

The end-results of these methods are reduced chain-scission and free-radical concentration, improved mechanical properties, improved oxidation resistance and enhanced wear resistance.

## BRIEF DESCRIPTION OF THE DRAWINGS

FIG. 1 shows the IZOD impact strengths of test samples 1, 3, 4, 6, 7, 8 and 9 of Table I;
FIG. 2 shows the average IZOD impact strengths of test sample of FIG. 1 before and after the HIPing process.

## DETAILED DESCRIPTION

Abbreviations used in this application are as follows:

UHMW - ultra-high molecular weight
UHMWPE - ultra-high molecular weight polyethylene
HMW - high molecular weight
HMWPE - high molecular weight polyethylene

This disclosure provides a method for improving the impact strength of a polymer by using a Hot Isostatic Pressing step before or after crosslinking using radiation and then thermally treating the resulting polymer.

The method as disclosed herein utilizes at least two separate irradiations for crosslinking a polymer followed by a like number of thermal treatments to decrease the free radicals to produce either a treated fully formed or a preformed polymeric composition. The term "preformed polymeric composition" or "preform" means that the polymeric composition is not in a final desired shape or form (i.e., not a final product). For example, where the final product of the preformed polymeric composition is an acetabular cup, the at least two irradiations and thermal treatments of the polymer could be performed on a pre-acetabular cup shape, such as when the preformed polymeric composition is in the form of a solid bar or block. Of course, the process of the present invention could be applied to a fully formed implant if the process is done with the implant in an oxygen reduced atmosphere.

In the method as disclosed herein, the wear resistance of a polymer is improved by crosslinking. The crosslinking can be achieved by various methods known in the art, for example, by irradiation from a gamma radiation source or from an electron beam, or from an X-ray radiation, or by photo crosslinking. The preferred method for crosslinking the polymer is by gamma irradiation. The polymer is preferably crosslinked in the form of an extruded bar or molded block.

In the preferred method, the crosslinked polymer is subjected to thermal or heat treatment such as by annealing (i.e. heated above at or below the melting temperature of the crosslinked polymer about 135°C) to produce the preformed polymeric composition. Alternately the heat treatment may be above the melt (between approximately 130° and 190°C).

High molecular weight (HMW) and ultra-high molecular weight (UHMW) polymers are preferred, such as HMW

polyethylene (HMWPE), UHMW polyethylene (UHMWPE), and UHMW polypropylene. HMW polymers have molecular weights ranging from about $10^5$ grams per mole to just below $10^6$. UHMW polymers have molecular weights equal to or higher than $10^6$ grams per mole, preferably from $10^6$ to about 107. The polymers are generally between about 400,000 grams per mole to about 10,000,000 and are preferably polyolefinic materials.

For implants, the preferred polymers are those that are wear resistant and have exceptional chemical resistance. UHMWPE is the most preferred polymer as it is known for these properties and is currently widely used to make acetabular cups for total hip prostheses and components of other joint replacements. Examples of UHMWPE are those having molecular weight ranging from about 1 to 8x$10^6$ grams per mole, examples of which are: GUR 1150 or 1050 (Hoechst-Celanese Corporation, League City, Tex.) with a weight average molecular weight of 5 to 6x$10^6$ grams per mole; GUR 1130 with a weight average molecular weight of 3 to 4x$10^6$; GUR 1120 or 1020 with a weight average molecular weight of 3 to 4x$10^6$; RCH 1000 (Hoechst-Celanese Corp.) with a weight average of molecular weight of 4x$10^6$ and HiFax 1900 of 2 to 4x$10^6$ (HiMont, Elkton, Md.). Historically, companies which make implants have used polyethylenes such as HIFAX 1900, GUR 1020, GUR 1050, GUR 1120 and GUR 1150 for making acetabular cups.

Sterilization Methods: All polymeric products must be sterilized by a suitable method prior to implanting in the human body. For the formed crosslinked and thermally treated polymeric compositions (i.e., the final products) of the present invention, it is preferable that the products be sterilized by a non-radiation based method, such as ethylene oxide or gas plasma, in order not to induce additional crosslinking free radicals and/or oxidation of the previously treated preformed polymeric composition. Compared to radiation sterilization, a non-radiation sterilization method has a minor effect on the other important physical characteristics of the product.

The degree of crystallinity can be determined using methods known in the art, e.g. by differential scanning calorimetry (DSC), which is generally used to assess the crystallinity and melting behavior of a polymer. Wang, X. & Salovey, R., J. App. Polymer Sci., 34:593-599 (1987).

Wide-angle X-ray scattering from the resulting polymer can also be used to further confirm the degree of crystallinity of the polymer, e.g. as described in Spruiell, J.E., & Clark, E.S., in "Methods of Experimental-Physics," L. Marton & C. Marton, Eds., Vol. 16, Part B, Academic Press, New York (1980). Other methods for determining the degree of crystallinity of the resulting polymer may include Fourier Transform Infrared Spectroscopy (FTIR), e.g., as described in "Fourier Transform Infrared Spectroscopy And Its Application To Polymeric Materials," John Wiley and Sons, New York, U.S.A. (1982)} and density measurement (ASTM D1505-68). Measurements of the gel content and swelling are generally used to characterize crosslink distributions in polymers; the procedure is described in Ding, Z.Y., et al., J. Polymer Sci., Polymer Chem., 29:1035-38 (1990). FTIR can also be used to assess the depth profiles of oxidation as well as other chemical changes such as unsaturation {Nagy, E.V. & Li, S., "A Fourier transform infrared technique for the evaluation of polyethylene orthopedic bearing materials," Trans. Soc. for Biomaterials, 13:109 (1990); Shinde, A. & Salovey, R., J. Polymer Sci., Polm. Phys. Ed., 23:1681-1689 (1985) } .

This disclosure also presents a process for making implants using the preformed polymeric composition of the present disclosure. The preformed polymeric composition may be shaped, e.g., machined, into the appropriate implants using methods known in the art. Preferably, the shaping process, such as machining, removing the oxidized surface of the composition.

The preformed polymeric compositions as disclosed herein can be used in any situation where a polymer, especially UHMWPE, is called for, but especially in situations where high wear resistance is desired. More particularly, these preformed polymeric compositions are useful for making implants.

This disclosure presents implants that are made with the above preformed polymeric compositions or according to the methods presented herein. In particular, the implants are produced from preformed polymeric composition made of UHMWPE irradiated and crosslinked at least twice each time followed by annealing and then removing the oxidized surface layer and then fabricating into a final shape. The preformed polymeric composition of the present disclosure can be used to make the acetabular cup, or the insert or liner of the cup, or trunnion bearings (e.g. between the modular head and the hip stem). In the knee joint, the tibial plateau (femoro-tibial articulation), the patellar button (patello-femoral articulation), and/or other bearing components, depending on the design of the artificial knee joint. These would include application to mobile bearing knees where articulation between the tibial insert and tibial tray occurs. In the shoulder, the process can be used in the glenoid component. In the ankle joint, the preformed polymeric composition can be used to make the talar surface (tibiotalar articulation) and other bearing components. In the elbow joint, the preformed polymeric composition can be used to make the radio-humeral joint, ulno-humeral joint, and other bearing components. In the spine, the preformed polymeric composition can be used to make intervertebral disk replacement and facet joint replacement. The preformed polymeric composition can also be made into temporo-mandibular joint (jaw) and finger joints. The above are by way of example, and are not meant to be limiting.

Free radicals generated during an irradiation step should be reduced to an acceptable level by annealing before exposure to oxygen. The portion of the material from which the implant is made contains free radicals and if it is exposed to air or other oxidants after the manufacturing process, oxidation will occur. The bulk portion of the polymer from which the implant is to be made should be annealed at an elevated temperature while out of contact with oxygen for a prescribed

time. This is because the rate of free radical reactions (reactions 10 through 12) increases with increasing temperature, according to the following general expressions:

$$\frac{dr\cdot}{dt} = k_1 [r\cdot] \text{ and } \frac{dP\cdot}{dt} = k_2 [P\cdot] \qquad (13)$$

Compared to room temperature, an elevated temperature not only increases the reaction rate constants, $k_1$ and $k_2$, but also helps free radicals r· and P· to migrate in the plastic matrix to meet other neighboring free radicals for cross-linking reactions. In general, the desired elevated temperature is between room temperature to below the melting point of the polymer. For UHMWPE, this temperature range is between about 25°C and about 140°C. It is to be noted that the higher the temperature used, the shorter the time period needed to combine free radicals. Additionally, due to the high viscosity of a UHMWPE melt, the formed UHMWPE often contains residual (internal) stress caused by incomplete relaxation during the cooling process, which is the last step of the forming process. The annealing process described herein will also help to eliminate or reduce the residual stress. A residual stress contained in a plastic matrix can cause dimensional instability and is in general undesirable.

In the preferred embodiment, the sequential irradiation followed by sequential annealing after each irradiation is performed in air on a preform such as an compressed block, bar or compression molded sheet made from polyethylene and preferably UHMWPE. Obviously, the final sequential annealing must take place prior to the bulk material of the final part or implant being exposed to air. Normally, it takes at least seven days for atmospheric oxygen to diffuse through the outer layer of polyethylene and deeply enough into rod, bar or sheet to effect the bulk polyethylene forming the final part. Therefore, the last annealing in the sequence preferably should take place prior to the time required for the oxygen to diffuse deeply into the rod. Of course, the more material which must be machined off to reach the finished part, the longer one can wait for the completion of the sequential irradiation and annealing process.

If the sequential irradiation/annealing process is performed on a final product, such as an acetabular cup, after machining, the polymeric component is preferably packaged in an air tight package in an oxidant-free atmosphere, i.e. less than 1% volume by volume of oxygen. Thus, all air and moisture must be removed from the package prior to the sealing step. Machines to accomplish this are commercially available, such as from Orics Industries Inc., College Point, New York, which flush the package with a chosen inert gas, vacuum the container, flush the container for the second time, and then heat seal the container with a lid. In general, less than 0.5% (volume by volume) oxygen concentration can be obtained consistently. An example of a suitable oxidant impermeable (air tight) packaging material is polyethylene terephthalate (PET). Other examples of oxidant impermeable packaging material is poly(ethylene vinyl alcohol) and aluminum foil, whose oxygen and water vapor transmission rates are essentially zero. All these materials are commercially available. Several other suitable commercial packaging materials utilize a layer structure to form a composite material with superior oxygen and moisture barrier properties. An example of this type is a layered composite comprised of polypropylene/poly (ethylene vinyl alcohol)/polypropylene.

With a final product, following each irradiation step, the heat treatment or annealing step should be performed while the implant is out of contact with oxygen or in an inert atmosphere and at an elevated temperature to cause free radicals to form cross-links without oxidation. If proper packaging materials and processes are used and oxidant transmission rates are minimal, then the oxidant-free atmosphere can be maintained in the package and a regular oven with air circulation can be used for heat treatment after sterilization. To absolutely ensure that no oxidants leak into the package, the oven may be operated under a vacuum or purged with an inert gas. In general, if a higher temperature is used, a shorter time period is required to achieve a prescribed level of oxidation resistance and cross-linking. In many cases, the relationship between the reaction temperature and the reaction rate follows the well-known Arrhennius equation:

$$k_1 \text{ or } k_2 = A * \exp(-\Delta H/RT) \qquad (14)$$

where $k_1$ and $k_2$ are reaction rate constants from reactions 13 and 14
A is a reaction dependent constant
$\Delta H$ is activation energy of reaction
T is absolute temperature (K)
R is the universal gas constant.

It is very important to ensure that the number of free radicals has been reduced to a minimal or an accepted level by the heat treatment. This is because the presence of an oxidant causes not only the oxidation of pre-existing free radicals, but also the formation of new free radicals via reactions 2 through 7. When the number of free radicals

grows, the extent of oxidation and the oxidation rate will increase according to the following equations:

$$\frac{dr\cdot}{dt} = k_3\ [r\cdot][O_2] \quad \text{and} \quad \frac{dP\cdot}{dt} = k_4\ [P\cdot][0_2] \qquad (15)$$

Where free radicals r· and P· can grow in number in the presence of oxidants and in turn increase the oxidation rates. It is also to be noted that the oxidation reaction rate constants $k_3$ and $k_4$ increase with increasing temperature, similar to $k_1$ and $k_2$. Therefore, to determine if a certain level of residual free radicals is acceptable or not, it is required to evaluate specific material properties after the plastic sample is stored or aged at the application temperature for a time period which is equal to or longer than the time period intended for the application of the plastic component. An alternative to the method to assess the aging effect is to raise the aging temperature of the plastic sample for a shorter time period. This will increase the reaction rate constants $k_3$ and $k_4$ significantly and shorten the aging time. It has been found that an acceptable level of residual free radicals is 1.0 x $10^{17}$/g for UHMWPE use for orthopedic implants.

The method as disclosed herein uses sequential thermal treatment under pressure (HIPing) to make a homogenized, consolidated UHMWPE for medical implants.

The sequential thermal treatment consists of a low temperature melting, ranging between 150-170° C, for 8 hours, annealing at 130°C for 2-10 hours, preferably 8 hours. Either treatment is followed by cooling to or below 50° C at a rate of equal to or less than 2° C/min, preferably less than 0.07° C/min. This thermal treatment is done while the preform is under pressure in argon.

The low temperature melting decrystallizes the consolidation polyethylene and eliminates the non-uniformity of the crystalline microstructure generated during consolidation. The subsequent annealing with the controlled cooling provides an optimal environment for polyethylene molecules to recrystallize to achieve ideal physical and mechanical properties.

The mechanical properties of non-cross-linked material after thermal treatment under pressure are not significantly changed. The effect of thermal treatment is observed after cross-linking.

The thermal and pressure treatments discussed above could be applied to any consolidated UHMWPE preform, the consolidation method including but not limited to, compression molding, extrusion, injection molding, and direct compression molding.

The thermal and pressure treatment could be conducted in an oven or in a pressurized vessel. The pressure applied should be no more than 30,000 psi, i.e. 207 Mpa but above 2,000 psi, i.e. 14 MPa.

The thermal treatment could be applied to non-cross-linked UHMWPE or cross-linked UHMWPE.

Twelve examples of consolidated GUR 1020 (extruded rod) where treated as set forth in Table I.

HIPing Matrix Starting material: virgin and sequentially cross-linked PE blocks followed by hot isostatic pressing as per Table I samples 1-12 followed by sequential cross-linking as in Example I

Table I

| Sample | | #1 | #2 | #3 | #4 | #5 | #6 |
|---|---|---|---|---|---|---|---|
| Heating rate (oC/min) | | - | - | - | - | - | 5 |
| Holding Temp (°C) | Below anneal | 0 | - | - | - | - | - |
| | Anneal | - | 130 | 130 | - | - | 130 |
| | Remelt | - | - | - | 170 | 170 | - |
| Holding pressure (psi) | Low | - | 2,000 | - | 2,000 | - | 2,000 |
| | Medium | - | - | 15,000 | - | 15,000 | - |
| | High | 30,000 | - | - | - | - | - |
| Holding time (hr) | | 8 | 8 | 8 | 8 | 8 | 8 |
| Cooling | | Immediate Release to room temperature | Immediate Release to room temperature | Immediate Release to room temperature | Immediate Release to room Temperature | Immediate Release to room temperature | 0.07°C/min from 130oC to 75oC under pressure |

| | | #7 | #8 | #9 | #10 | #11 | #12 |
|---|---|---|---|---|---|---|---|
| Heating rate (°C/min) | | 5 | 6 | 7 | | | |
| Holding Temp (°C) | Below anneal | - | - | - | | | |
| | Anneal | - | 130 | - | | | |
| | Remelt | 170 | - | 170 | 150 | 163 | 163/95 |
| Holding pressure (psi) | Low | 2,000 | - | - | 2,000 | 2,000 | 2,000 |
| | Medium | - | 15,000 | 15,000 | | | |
| | High | - | - | - | | | |
| Holding time (hr) | | | 8 | 8 | 8 | 8 | 8/4 |
| Cooling | | 0.07°C/min from 170°C to 75°C under pressure | 0.07°C/min from 130°C to 75°C under pressure | 0.07°C/min from 170°C to 75°C under pressure | 0.07°C/min from 150°C to 75°C under pressure | 0.07°C/min from 163°C to 75°C under pressure | 0.7°C/min from 163°C to 95°C and 0.2°C/min from 95°C to 75°C under pressure |

Table II shows the results for seven test samples (1, 3, 4, 6, 7 and 9) which best illustrate the improved impact strengths of the method of the present invention. A graph of the results are shown in Fig. 1.

Table II

| (Summary of seven samples from Table I) Sequentially cross-linked (GUR 1020) | | | | | | | | |
|---|---|---|---|---|---|---|---|---|
| Sample ID | Heating Rate | (°C/ Holding Temp (°C) | Holding Pressure (PSI) | Holding Time (hrs) | Cooling | Izod Strength | Izod Stdev | Before-HIPing Izod |
| 1 (30,000 psi RT) | - | 0 | 30000 | 8 | Immediate Release | 58 | 0.7 | 58 |
| 3 (15,000 psi anneal) | - | 130 | 15000 | 8 | Immediate Release | 57 | 0.7 | 58 |
| 4 (20,000 psi remelt) | - | 170 | 2000 | 8 | Immediate Release | 71 | 1.2 | 58 |
| 6 (2,000 psi, anneal) | 5 | 130 | 2000 | 8 | 0.07°C/min from 130°C to 75°C under pressure | 56 | 0.7 | 57 |
| 7 (2,000 psi, anneal) | 5 | 170 | 2000 | 8 | 0.07°C/min from 130°C to 75°C under pressure | 63 | 0.8 | 56 |
| 8 (15,000 psi RT) | 6 | 130 | 15000 | 8 | 0.07°C/min from 130°C to 75°C under pressure | 56 | 1.3 | 58 |

(continued)

| (Summary of seven samples from Table I)<br>Sequentially cross-linked (GUR 1020) | | | | | | | | |
|---|---|---|---|---|---|---|---|---|
| Sample ID | Heating Rate | (°C/ Holding Temp (°C) | Holding Pressure (PSI) | Holding Time (hrs) | Cooling | Izod Strength | Izod Stdev | Before-HIPing Izod |
| 9 (15,000 psi remelt) | 7 | 170 | 15000 | 8 | 0.07°C/min from 130°C to 75°C under pressure | 60 | 3.2 | 57 |

Example I (Condition #7 above)

Ultra-high molecular weight polyethylene preform, from a compression molded block, is hot isostatically pressed at a pressure of 2,000 psi, i.e. 14 MPa, and a temperature of 170°C for 8 hours and then cooled at a rate of 0.07°C/min after irradiated sequentially for a sufficient time for an accumulated incremental dose of between 20 to 50 kGy. After each irradiation step, the block is annealed in air preferably at a temperature below its melting point, preferably at less than 135°C and more preferably between 110°C and 130°C. The block is then allowed to cool to room temperature. The irradiation and annealing steps are repeated two or more times so that the total radiation dose is between 50 to 150 kGy. In this example I, the block is irradiated for a total dose of 30 kGy and then annealed at 130°C for 24 hours, allowed to cool to room temperature and sit for 3 days and then reirradiated for a dose of 30 kGy (a total dose of 60 kGy) again annealed at 130°C for 24 hours, allowed to cool at room temperature and sit for an additional 3 days and then irradiated a third time with a 30 kGy dose (for a total of 90 kGy) and again annealed at 130°C for 24 hours. Impact strength of the block is tested under ASTM standard D256 to determine the IZOD Pendulum Impact Resistance. The IZOD results for sample 7 are shown in Table III and shown in the graph of Fig. 2.

Table III

| Thermal treatment post sequential cross-linking and annealing (HIPing) | Yes | No |
|---|---|---|
| Izod impact strength ($kJ/m^2$) | $63 \pm 0.8$ | $56 \pm 1.1$ |

A similar result occurs when the Hiping is performed prior to sequential cross-linking.

| | Pre-crosslinking property | | Post-crosslinking property | |
|---|---|---|---|---|
| HIPing | Yes | No | Yes | No |
| Izod impact strength ($kJ/m^2$) | $147 \pm 2$ | $145 \pm 2$ | $62 \pm 1$ | $59 \pm 1$ |
| Ultimate tensile strength (MPa) | $50 \pm 9$ | $52 \pm 14$ | $50 \pm 12$ | $62 \pm 6$ |
| Tensile yield strength (MPa) | $24 \pm 0$ | $24 \pm 0$ | $24 \pm 0$ | $24 \pm 0$ |
| Tensile elongation (%) | $370 \pm 49$ | $351 \pm 69$ | $251 \pm 38$ | 28114 |

Example 2:

Compression molded GUR1020 was thermal treated at 150° C for 8 hours before the first irradiation step of the sequential cross-linking and then sequentially irradiated and annealed. The Izod impact strength and tensile properties were measured. The Izod impact strength of thermal treated material did not differ significantly from the non-thermal treated material before cross-linking. However, the Izod impact strength of thermal treated and sequentially cross-linked and annealed material increased while the tensile properties remained similar.

Table IV

| The test resin (150° C for 8 hours) | Pre-crosslinking property | | Post-crosslinking property | |
|---|---|---|---|---|
| Thermal treatment | Yes | No | Yes | No |
| Izod impact strength (kJ/m$^2$) ASTM D256 | 146.7 ± 2.0 | 145.1 ± 1.6 | 63.8 ± 1.2 | 59.7 ± 0.8 |
| Ultimate tensile strength (MPa) ASTM D638 | - | - | 60 ± 5 | 60 ± 5 |
| Tensile yield strength (MPa) ASTM D638 | - | - | 24 | 24 |
| Tensile elongation (%) | - | - | 277 | 269 |

The above example can also be applied to compression molded sheet with, for example, a tibial component being manufactured out of the sequentially irradiated and annealed material. What is important is that a two-step consolidation is applied. The first consolidation is from the GUR 1020 resin to form a rod, bar or plate. The second consolidation is a hot isostatic pressing before or after irradiation and annealing or remelting.

Although the invention herein has been described with reference to particular embodiments, it is to be understood that these embodiments are merely illustrative of the principles and applications of the present invention.

## Claims

**1.** A method for making an ultra-high molecular weight polyethylene (UHMWPE) medical implant comprising:

obtaining a preform consolidated from UHMWPE resin;
thereafter hot isostatically pressing a UHMWPE preform at a temperature between 150°C and 170°C for 8 hours at a pressure of between 14 and 207 MPa (2000 and 30.000 psi);
thereafter irradiating the UHMWPE perform in a solid state at a total radiation dose of 20 to 100 kGy;
thereafter heating the irradiated UHMWPE preform to a temperature of 110°C to 130°C for between 2 to 10 hours;
cooling the irradiated and heated preform to or below 50°C; and
thereafter forming a medical implant from the preform.

**2.** The method as set forth in claim 1, wherein the UHMWPE perform is consolidated by a method selected from the group consisting of direct compression molding, extrusion and injection molding.

**3.** The method of claim 1 or claim 2, wherein the UHMWPE perform is irradiated at least two times and heated after each irradiation followed by cooling after each heating.

**4.** The method as set forth in any one of claims 1-3, wherein the cooling to or below 50°C is done at a rate equal to or less than 2°C/min.

**5.** The method as set forth in claim 4, wherein the rate is less than 0.07°C/min.

## Patentansprüche

**1.** Verfahren zur Herstellung eines medizinischen Implantats aus ultrahochmolekularem Polyethylen (UHMWPE), umfassend:

Erhalten einer Vorform, die aus UHMWPE-Harz konsolidiert wurde;
danach isostatisches Heißpressen einer UHMWPE-Vorform bei einer Temperatur zwischen 150 °C und 170 °C für 8 Stunden bei einem Druck zwischen 14 und 207 MPa (2000 und 30000 psi);
danach Bestrahlung der UHMWPE-Vorform im festen Zustand bei einer Gesamtstrahlungsdosis von 20 bis 100 kGy;

danach Erwärmen der bestrahlten UHMWPE-Vorform auf eine Temperatur von 110 °C bis 130 °C für 2 bis 10 Stunden;
Kühlen der bestrahlten und erwärmten Vorform auf oder unter 50 °C;
und
danach Bilden eines medizinischen Implantats aus der Vorform.

**2.** Verfahren nach Anspruch 1, wobei die UHMWPE-Vorform durch ein Verfahren konsolidiert wird, das aus der Gruppe ausgewählt ist, die aus dem direkten Pressformen, der Extrusion und dem Spritzgießen besteht.

**3.** Verfahren nach Anspruch 1 oder Anspruch 2, wobei die UHMWPE-Vorform mindestens zweimal bestrahlt und nach jeder Bestrahlung erwärmt wird, gefolgt von einer Kühlung nach jeder Erwärmung.

**4.** Verfahren nach einem der Ansprüche 1-3, wobei das Kühlen auf oder unter 50 °C mit einer Geschwindigkeit von gleich oder weniger als 2 °C/min erfolgt.

**5.** Verfahren nach Anspruch 4, wobei die Geschwindigkeit weniger als 0,07 °C/min beträgt.

## Revendications

**1.** Procédé de fabrication d'un implant médical en polyéthylène à ultra haut poids moléculaire (UHMWPE) comprenant :

l'obtention d'une préforme consolidée à partir de résine UHMWPE ;
puis la compression isostatique à chaud d'une préforme en UHMWPE à une température entre 150°C et 170°C pendant 8 heures à une pression entre 14 et 207 MPa (2000 et 30 000 psi);
puis l'irradiation de la préforme en UHMWPE à l'état solide à une dose de rayonnement totale de 20 à 100 kGy ;
puis le chauffage de la préforme en UHMWPE irradiée à une température de 110°C à 130°C pendant 2 à 10 heures ;
le refroidissement de la préforme irradiée et chauffée à une température de 50°C ou en dessous ; et
la formation ensuite d'un implant médical à partir de la préforme.

**2.** Procédé selon la revendication 1, dans lequel la préforme en UHMWPE est consolidée par un procédé choisi dans le groupe constitué du moulage par compression directe, de l'extrusion et du moulage par injection.

**3.** Procédé selon la revendication 1 ou la revendication 2, dans lequel la préforme en UHMWPE est irradiée au moins deux fois et chauffée après chaque irradiation suivie d'un refroidissement après chaque chauffage.

**4.** Procédé selon l'une quelconque des revendications 1 à 3, dans lequel le refroidissement à 50°C ou en dessous est effectué à une vitesse égale ou inférieure à 2°C/min.

**5.** Procédé selon la revendication 4, dans lequel la vitesse est inférieure à 0,07 °C/min.

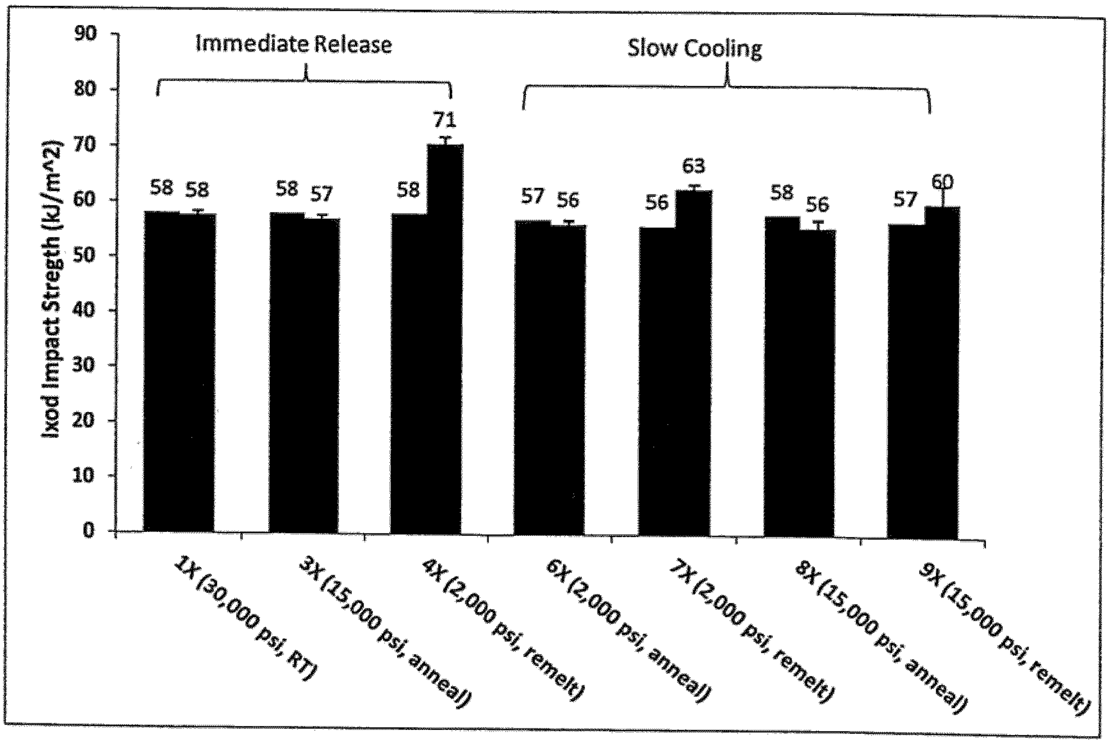
Ixod Impact Stregth (kJ/m^2)
90
80
70
60
50
40
30
20
10
0
Immediate Release
Slow Cooling
58 58
58 57
58 71
57 56
56 63
58 56
57 60
1X (30,000 psi, RT)
3X (15,000 psi, anneal)
4X (2,000 psi, remelt)
6X (2,000 psi, anneal)
7X (2,000 psi, remelt)
8X (15,000 psi, anneal)
9X (15,000 psi, remelt)

FIG. 1

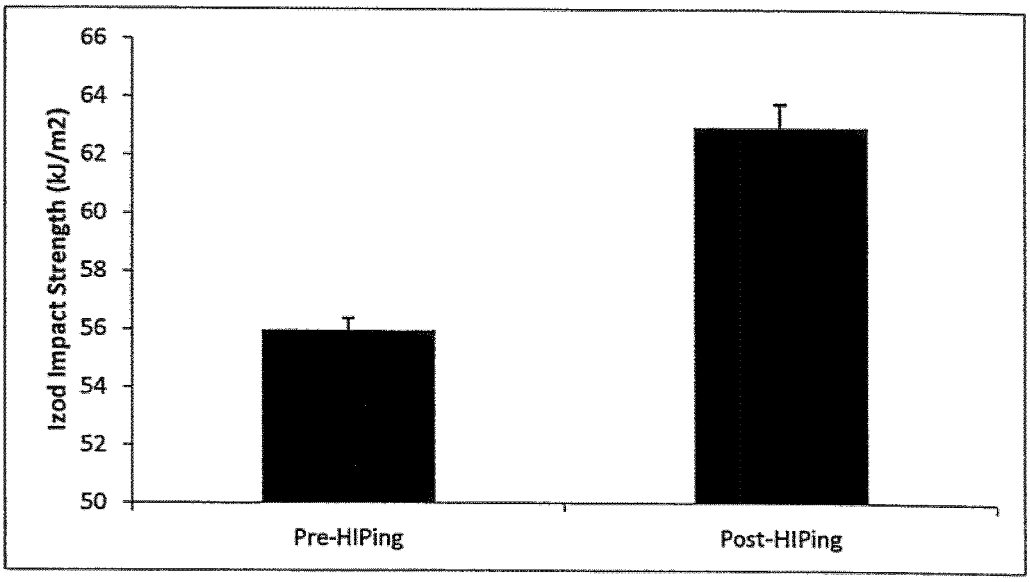
66
64
62
60
58
56
54
52
50
Izod Impact Strength (kJ/m2)
Pre-HIPing
Post-HIPing

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5414049 A, Sun
- US 6168626 B, Hyon
- US 6228900 B, Salovey
- US 6316158 B, Saum
- EP 017752281 A
- US 5037928 A
- GB 2180815 A
- US 5153039 A
- US 5160464 A
- EP 03253363 A
- EP 1369135 A1
- US 2008059909 W
- WO 2008124825 A2
- US 7517917 B
- US 7714036 B
- US 8030370 B
- US 8324291 B


### Non-patent literature cited in the description


- Radiation Effects on Polymers. American Chemical Society, 1991
- **WANG, X. ; SALOVEY, R.** *J. App. Polymer Sci.,* 1987, vol. 34, 593-599
- **SPRUIELL, J.E. ; CLARK, E.S.** Methods of Experimental-Physics. Academic Press, 1980, vol. 16
- Fourier Transform Infrared Spectroscopy And Its Application To Polymeric Materials. John Wiley and Sons, 1982
- **DING, Z.Y. et al.** *J. Polymer Sci., Polymer Chem.,* 1990, vol. 29, 1035-38
- **NAGY, E.V. ; LI, S.** A Fourier transform infrared technique for the evaluation of polyethylene orthopedic bearing materials. *Trans. Soc. for Biomaterials,* 1990, vol. 13, 109
- **SHINDE, A. ; SALOVEY, R.** J. Polymer Sci. *Polm. Phys. Ed.,* 1985, vol. 23, 1681-1689